# EUROPEAN PATENT APPLICATION

(11) **EP 1 640 717 A1**
(43) Date of publication of application: **29.03.2006**
(21) Application number: 04292315.1
(22) Date of filing: 28.09.2004
(51) Int. Cl.: G01N 33/50

(54) **Method and kits for in vitro identification of cells, particularly lymphoid cells, capable of establishing interactions with target cells and biological applications thereof**

(71) Applicant: INSTITUT NATIONAL DE LA SANTE ET DE LA RECHERCHE MEDICALE (INSERM), 75654 Paris Cédex 13 (FR)
(72) Inventor: Fournie, Jean-Jacques, 31450 Corronsac (FR); Poupot, Mary, 31280 Aigrefeuille (FR); Pont, Frédéric, 31300 Toulouse (FR)
(74) Representative: Peaucelle, Chantal

(57) **Abstract**

The invention relates to a method for *in vitro* identifying the living cells of a sample to be tested capable of interacting through trogocytosis with targets, wherein the living cells consist of or comprise as a major part lymphoid cells and the target cells are labelled, said method comprising detecting the label transferred from the targets to the living cells, to identify the effector cells. It also relates to the kits for carrying out said method.

Applications, particularly, in diagnostic, therapeutics and research.

## Description

The invention relates to a method and kits for *in vitro* identification of cells, particularly lymphoid cells, capable of establishing interactions with target cells. It also relates to their biological applications, particularly for diagnostic, research or therapeutic uses.

The cells which are able to establish such interactions will be designated hereinafter "effector cells".

Several reports relate to the transfer of surface molecules from antigens to lymphocytes through immunological synapses (see e.g.Huang *et al,* 1999; Hudrisier and Bongrand, 2002; Joly and Hudrisier, 2003; Hudrisier *et al,* 2001;Tabiasco *et al,* 2003; Tabiasco *et al,* 2002; Espinosa *et al* 2002; Batista *et al,* 2001;Batista *et al,* 2000). This transfer phenomenon has been designated by the term "trogocytosis".

The inventors have now found that the trogocytosis phenomenon could advantageously be used for the identification of effector cells in cell population(s).

It is then an object of the invention to provide a method for the *in vitro* identification of living effector cells from a sample,capable of interacting through trogocytosis with targets.

Another object of the invention is to provide kits to carry out said method.

According to still another aspect, the invention relates to the use of said method and/or kits for research, and/or for diagnostic, and/or therapeutical applications.

The invention relates to a method for *in vitro* identifying the living cells from a sample to be tested capable of interacting through trogocytosis with targets, wherein the living cells consist of or comprise as a major part lymphoid cells and the target cells are labelled, said method comprising detecting the label transferred from the target cells to the living cells, to identify the effector cells.

By using said method, it is then possible to specifically identify, from a complex cellular mixture, the cells responsible for interactions with target cells.

More particularly, the *in vitro* identification method of the invention comprises
- inserting a fluorescent label at the membrane surface of the target cells,
- co-incubating the fluorescent labelled target cells with said living cells, under conditions enabling the establishment of the interactions between both cellular types in case of trogocytosis, and
- identifying the effector cells, i.e. cells having established such interactions, and
- quantifying and comparing the amount of transferred label between different types or subsets of living cells, if desired.

Such a method advantageously enables a rapid identification and quantification of the great number of interactions between living cells and their targets.

The target cells correspond to one or several types of mixed cells interacting simultaneously. For example, the target cells are cancer cells or cancer cell lines.

Alternatively, the target cells are not living cells, or are not cells. For example, they consist of a solid surface labelled with the fluorochrome, such as microbeads , and covered with a material susceptible to be recognized by the living cells.

According to the invention, the insertion of the label is usually carried out at room temperature and comprises adding a suspension of the target cells in an appropriate medium to a solution containing the label.

The labels for the target cells are preferably lipophilic fluorochrome, such as PKH67, or membrane protein labelled by an antibody conjugated to a lipophilic fluorochrome.

The living cells to be tested are usually extracted from biological samples of a patient. They correspond to one or more cell population(s) from different subsets, particularly from different lymphoid subsets.

The biological samples are for example blood samples collected in heparinized sterile tubes and the living cells extracted therefrom are PBMC or subsets populations of PBMC.

The co-incubation step is carried out during a period of time and under physiological conditions appropriate with the viability of the cells.

Satisfactory incubation conditions comprise co-culture of the target cells and the living cells to be tested during various times such as for example 1h in a culture medium, at physiological temperature, under humidified 5% CO₂ in air atmosphere.

The cells are co-cultured in culture supports, particularly the test tubes or single culture wells or flasks or multiwell culture plates.

To promote cell contacts, the supports, particularly the test tubes, or single culture wells, or flasks, or multiwell culture plates, are centrifuged.

The effector cells are then identified by their acquisition of the fluorescent label from the target cells.

For identification of the subsets of effector cells, the co-cultures are rinsed in a buffer to dissociate aggregates, and further labelled with specific antibodies conjugated to a fluorochrome distinct from that used to stain the targets, and more precisely with a different emission wavelength spectrum.

The quantification step of the dual fluorescence intensities on the effector cells is advantageously carried out by flow cytometry.

As disclosed in the examples, the multiparametric measurement of the acquired fluorescence from the target cells by the effector or each type of effectors cells, as identified by said specific conjugates, is a direct quantification of the interaction with the target.

Advantageously, several experiments using said method can be performed in parallel, by placing for example each type of targets cells or each type of effector cells to be tested, for example, in individual test tubes or wells or multiwell plates, and using an automated sampling and polychormatic flow cytometry analyzer, for example equipped with an automatic injector from multiwell plates apparatus.

Advantageously, multiple experiments using said method can be performed simultaneously or in parallel in the same culture well or flask or multiwell plates, by placing in each coculture, for example, several types of target cells, each of which are respectively labelled with distinct membrane fluorochromes, with several types of effector cells, identified further with specific antibodies conjugated to fluorochromes yet distinct from those used to stain the targets, and more precisely with a different emission wavelength spectra, and using an automated polychromatic flow cytometry analyser, for example equipped with an automated injector from mutliwell plates apparatus.

The invention also relates to kits for carrying out the method such as above defined.

Said kits comprise cell labels, particularly lipophilic fluorochromes for the labelling of cell targets and antibodies.

They also advantageously further comprise culture buffers and/or rinsing buffers.

According to another embodiment, the kits of the invention also comprise target cells and/or effector cells, and/or culture supports.

The invention thus provides means for the rapid detection and typing of a great number of living cells of a given phenotype interacting with synthetic or cell targets from bulk *in vitro* cell mixtures.

The method and kits of the invention are of great interest in several fields. As illustrated by the examples, they are more particularly useful for simultaneously identifying and comparing the cell-to-cell interactions of a whole sample of human blood with a broad panel of targets, such as a collection of distinct cell lines of human tumoral cells.

The invention also provide means to identify hybridoma producing target cell-specific monoclonal antibody against a defined antigen cell, here tested as the target.

It also enables to identify T or NK lymphocytes responsible for the specific responses to a cell target.

In the same way, haematopoietic cells responsible for an auto-immune interaction with an autologous target cell can be identified.

The invention thus also relates to the use of said method and/or kits as research or diagnostic tools, or for therapeutical applications, particularly in hematology, oncology, immunology, allergology, bacteriology, mycology/parasitology, toxicology/pharmacology and virology.

Other characteristics and advantages of the invention will be given in the following examples with reference to figures 1 to 5, which represent, respectively:
- Figure 1: interaction of freshly collected whole unseparated human PBMC with co-incubated Daudi cells identified by spontaneous trogocytosis,
- Figure 2: human PBMC subsets interacting with Daudi cells,
- Figure 3: the determination by tumor cells of the interactions with PBMC subsets,
- Figure 4: the simultaneous profiling of the *ex vivo* interaction of fresh PBMC with different cancer cell lines tested in parallel, and
- Figure 5: subtyping of peripheral γδ T lymphocytes interacting with co-incubated Karpas-299 cells.

### MATERIAL AND METHODS

### Blood samples

Fresh blood samples were collected from healthy, EBV+ adult volunteers on heparinized vacuum tubes at the Centre de Transfusion Sanguine de Midi-Pyrénées (Hopital Purpan, Toulouse). These samples were processed to isolate peripheral blood mononuclear cells by dilution 1: 2 in RPMI 1640 prior to centrifugation 1000xg , 10 minutes on Leucosep™ tubes Greiner bio-one, Frickenhausen, Ger) for collection of PBMC. The cells are then washed twice in RPMI 1640 prior to assays.

### Cell lines

All tumor cell lines were continuously maintained by culture at cell densities between 5-10x10⁵ cells/ml in RPMI 1640 with Glutamax-I (Life technologies, Paisley, Scotland), supplemented with 10% FCS (Invitrogen, Cergy Pontoise, France), 25 mM Hepes, 100 U/ml penicillin G, 100 µg/ml streptomycin and 1 mM sodium pyruvate.

All adherent cells were trypsinized (Gibco-InvitroGen, Cergy Pontoise) for 3 minutes at 37°C and washed in culture medium prior to labeling by PKH67 as specified. The viability of labelled targets was always superior to 90% in assays.

### Reagents

The fluorochromes PKH26 and PKH67 were purchased from Sigma-Aldrich (St Louis, MI) and used according to supplier's protocols.

In brief, 5x10⁶ pelleted cells were resuspended in 250 µl of manufacturer's diluent C (Sigma), and added to 250 µl of PKH solution (4 µM in diluent C) for 5 minutes at RT. The stainings were then stopped with 500 µl fetal calf serum prior to washing the cells three times with RPMI 1640. PKH-labeled cells were then assayed immediately for transfer, but remained viable in culture for up to 7 days while their fluorescence progressively diluted along with division.

The following reagents were purchased from Beckman-Coulter-Immunotech (Marseille,France): PE-conjugated monoclonal antibodies anti-CD20, anti-CD16, anti-pan TCR αβ, anti-pan TCRγδ, anti-CD4, anti-CD8 (1/50 dilution as recommended by suppliers); anti human Igµ (4 µg/ml) and GAM-PE conjugate (diluted 1/50) while isotype-matched Ig controls (Dako, Denmark) were used at 5 µg/ml.

### Staining with fluorochromes and flow cytometry

Cells were stained as described above, placed in co-cultures in 96-wells U-bottom tissue culture plates, and were done at a final concentration of 6x10⁵ cells in 100 µl of complete RPMI+10% FCS.

Culture plates were then centrifuged 1 minute at 700 rpm to promote cell contact and were left for 1hr at 37°C in humidified 5% CO₂.

After 0 and 60 minutes of co-culture, cells were then washed twice with PBS containing 0.5 mM EDTA for dissociating aggregates and analyzed by flow cytometry using a FACSCalibur™ and CELL Quest™ software (Becton-Dickinson, CAL), as described by Poupot et al, 2003. In each experiment, the PKH67 mfi values were obtained from 20,000 gated cells in each sample; experiments were repeated three to five times.

For pre-treatment of PKH-labeled cells, these were kept either with anti-Igµ (4µg/ml, 45 minutes at 4°C) or control Ig isotypes (10µg/ml, same conditions), mixed by gentle peleting, incubated for one hour at 37°C and then analyzed by flow cytometry.

When specified, the PBMC subsets were identified by the following mAbs: anti-CD20-PE (blood B lymphocytes), anti-CD 16-PE (NK cells), anti-pan TCR γδ-PE (γδ T lymphocytes), anti-pan TCR αβ-PE(αβ T lymphocytes), anti-CD4-PE and anti-CD8-PE.

### Graphical representation

The mfi values obtained for each transfer by flow cytometry are visualized by a graphical colour code computed with a software developed in-house which reads the text files and programed in PERL (http://www.perl.org/)on a PC running the linux RedHat 9.0 operating system (http://www.redhat.com/).

Briefly, the software reads the text files comprising each experimental mfi value, assigns to this data a grey level intensity by linear interpolation from the extreme mfi values and generates a portable pixmap file(PPM) in binary format of 256 grey levels. The file is then converted to TIF and coloured by applying a green lookup table (given in the figure). Each mfi is thus represented by a colored square and generates a TIF picture by bringing together all mfi values. In addition, mean values for each line and column are also generated and represented in parallel columns.

### Statistical analysis

All series of experimental fluorescence intensities were based on n = 10,000-20,000 events for which arithmetic means and variance were computed with the softwares CellQuest™ (Becton-Dickinson,CAL) and WinMDI 2.8(http://facs.scripps.edu/). Because the series of fluorescence intensities are not best described as normal distributions, their statistical comparison are done by non-parametric analysis by the Mann-Whitney rank sum test.

### RESULTS

### Tumor cell trogocytosis by bulk of human PBMC.

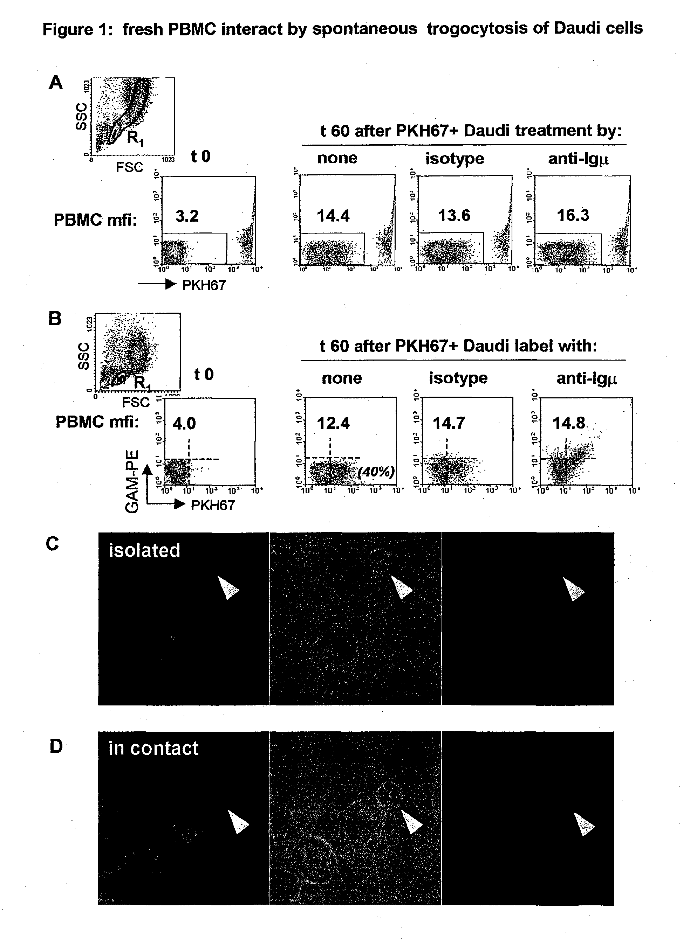

The results obtained with co-cultures of fresh PBMC with Daudi celles are given on Figures 1A and 1B, which represent:
A: PKH67 fluorescence of PBMC co-incubated for 0 and 60 minutes with PKH67+ Daudi cells (R1 gate) in the specified conditions (numbers indicate the PKH67mfi for boxes); and
B: the flow cytometry results which show simultaneous acquisition of Daudi-derived PKH67 and Igµ by co-incubated PBMC (gated in R1).

In these experiments, Daudi cells stably stained with the membrane fluorochrome PKH67 were co-incubated for one hour with bulk unstained PBMC in complete culture medium prior to FACS analysis.

Morphological gating by flow cytometry FSC/SSC parameters unambiguously discriminated small PBMC from the larger lymphoma cells detected as PKH67bright in FL1/FL2 dot plots.

Comparing the mean intensity of PKH67 fluorescence (referred below to as mfi) on gated PBMC at t0 and t60 minutes showed an increase upon co-culture, indicating acquisition of Daudi cells-derived PKH67 by PBMC.

Acquisition of a transmembrane protein from the lymphoma cell surface was measured to check that this observation reflected trogocytosis.

The Igµ of PKH67+ Daudi cells was labelled with specific mouse Ig and secondary PE-conjugated goat anti-mouse Ig, prior to measuring transfer as above.

Control experiments in which Daudi cells were either unlabelled, or labeled with isotype-matched control Ig alone, or labelled with mouse anti-Igµ alone, or with isotype control plus secondary PE-conjugated goat anti-mouse did not reveal any increase in PE fluorescence on the PBMC after one hour of co-culture (Fig. 1A).

Nevertheless the PBMC co-incubated with the PKH67+ Igµ-PE+ Daudi cells had acquired both of these fluorescences (Fig. 1B), demonstrating that PBMC which acquired PKH67 from tumor cells acquired as well their Igµ transmembrane protein.

Said results demonstrate that *in vitro* co-incubation enabled bulk PBMC's to interact with cancer cells through trogocytosis.

### Detection of subsets which trogocytose Daudi from bulk PBMC's.

After the trogocytosis, the co-culture was labelled with CD-specific PE-conjugates in parallel assays to determine whether this transfer was differentially mediated by a specific PBMC subset.

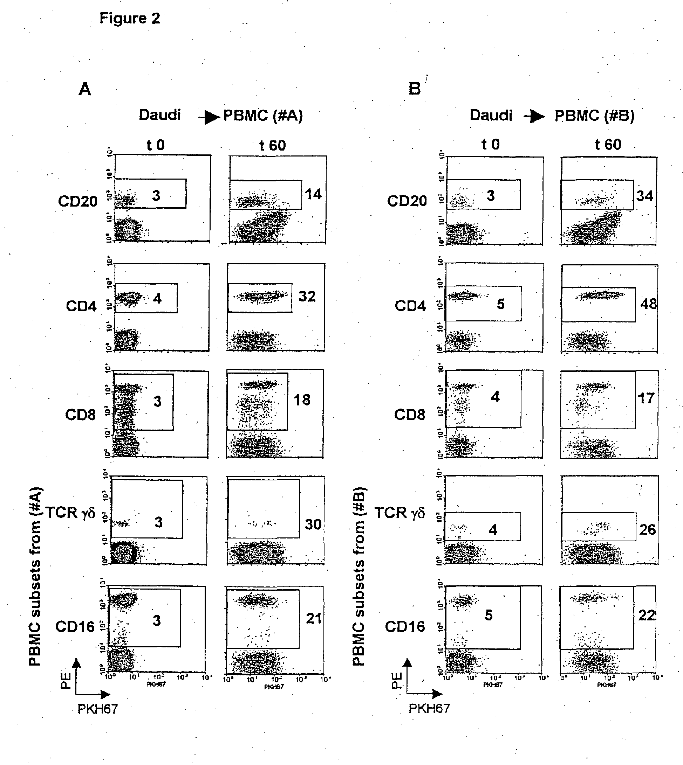

The markers used in the shown example were CD20 (B cells), CD4, CD8 (αβ T lymphocytes), pan-γδ TCR (γδ T lymphocytes) and CD16, here used to merely reflect NK cells.
The results are given in Figures 2A and 2B, illustrate the different abilities of PBMC from distinct human individuals to interact with a defined cancer cell tested here as target:
A: Differential trogocytosis of Daudi cells by the specified subsets of bulk PBMC freshly isolated from donor # A (numbers indicate the PKH67mfi for boxes). Each subset was gated using isotype control-PE conjugates (not shown).
B: Similar experiment with bulk PBMC from donor # B (representative results for n > 3 independent experiments.).

2D FACS analysis of gated PBMC showed that all five PBMC subsets had done trogocytosis from Daudi, although at intensities. Thes latter are not related to the respective abundance of the effector cell subset within the bulk of PBMC: CD20 cells (15 % of PBMC) had reached mfi of 14, CD4 cells (52 % of PBMC) : mfi 32, CD8 (20% of PBMC) : mfi 18, γδ T cells (1% of PBMC) : mfi 30 and CD 16 cells (19% of PBMC) : mfi 21 (Fig. 2A).

So each subset of PBMC had distinct interactions with the tumor cell. The same pattern of reactivity to Daudi cells was recovered within five successive tests of PBMC from donor #A collected and tested over a period of three months. So this interaction pattern is a stable feature for a defined individual and cancer cell line.

With another healthy EBV+ donor # B, the results differed slightly. While its CD8, γδ T and NK subsets scored similarly as those from donor # A, its B and CD4 lymphocytes interacted stronger with Daudi than those from donor #A (P<0,001 for both comparisons B-to-B and CD4-to-CD4).

An efficient interaction of B, CD4 T and γδ T cells from healthy EBV+ donors, when coincubated with the EBV+ Daudi Burkitt's lymphoma, was not unexpected. Although the HLA class I deficiency of Daudi cells could account for their ignorance by HLA class-I-restricted CD8 T lymphocytes, it determined their attack by γδ T cells. Such feature usually favors NK cell activation, this was not found here since peripheral NK cells are in resting state. In agreement with this assumption, with IL-2-activated PBMC from donor # A instead of freshly isolated cells, both γδ T and NK cells gave very strong trogocytosis of Daudi (mfi > 100).

So this assay not only detected the respective interactions of each PBMC subset with Daudi, and thereof defined an interaction pattern, but also pinpointed inter-individual differences for this pattern.

### Distinct subsets of PBMC trogocytose differently distinct tumor cell lines.

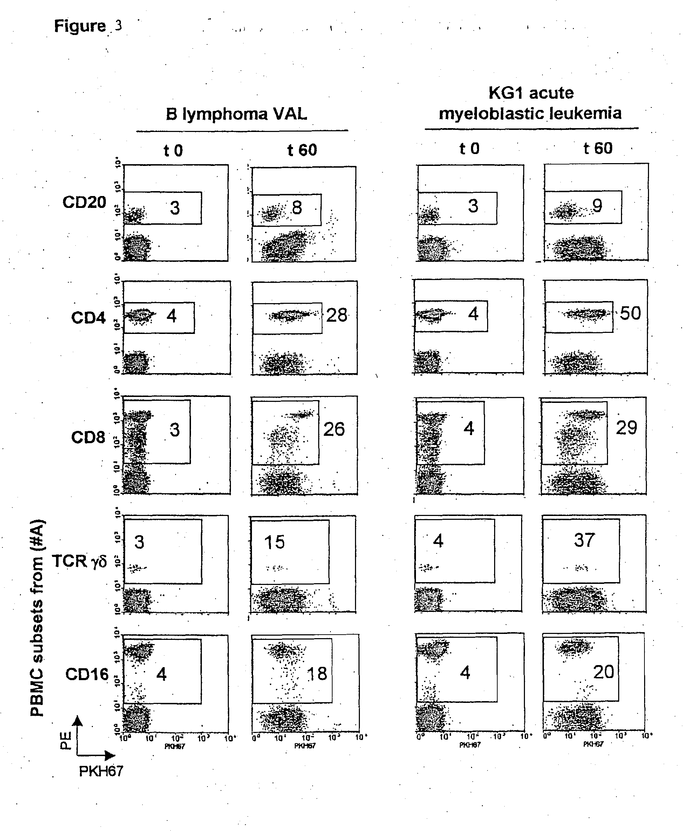

In other experiments, the interaction of the PBMC from donor #A with two other cell lines, VAL (a B cell lymphoma) and KG1 (an EBV- acute myeloblastic leukemia) was studied to evaluate whether the interactions were similar.
The results are given in Figure 3 which represents interactions of fresh PBMC subsets from donor # A co-incubated with either the PKH67+ lymphoma VAL (left) or with PKH67+ KG1 acute myeloblastic leukemia cells (right) (numbers indicate the PKH67mfi for boxes).

Although interactions with PBMC were detected again, they differred from those with Daudi. VAL was nearly ignored by the fresh B cells but strongly trogocytosed by both CD4 T and CD8 T lymphocytes.

With KG1 though, all CD4, CD8 and γδ T lymphocytes had mediated transfer. In addition, these interactions with KG1 were significantly stronger than with both VAL and Daudi B cell lymphoma.

So the cancer cell "target" influenced its interactions with each subset of freshly collected PBMC. This confirmed that the immunological scanning of cancer cells was not only determined by the PBMC, but also by the tumor cell phenotype.

### Profiling PBMC interactions with a panel of cancer cell lines

In view of the above results, the whole pattern of cell interactions involving the same subsets of PBMC from the same donor #A with a larger set of cancer cell lines tested as targets can be compared more extensively by the same procedure using parallel assay run using parallel co-cultures from multiwell culture plates.

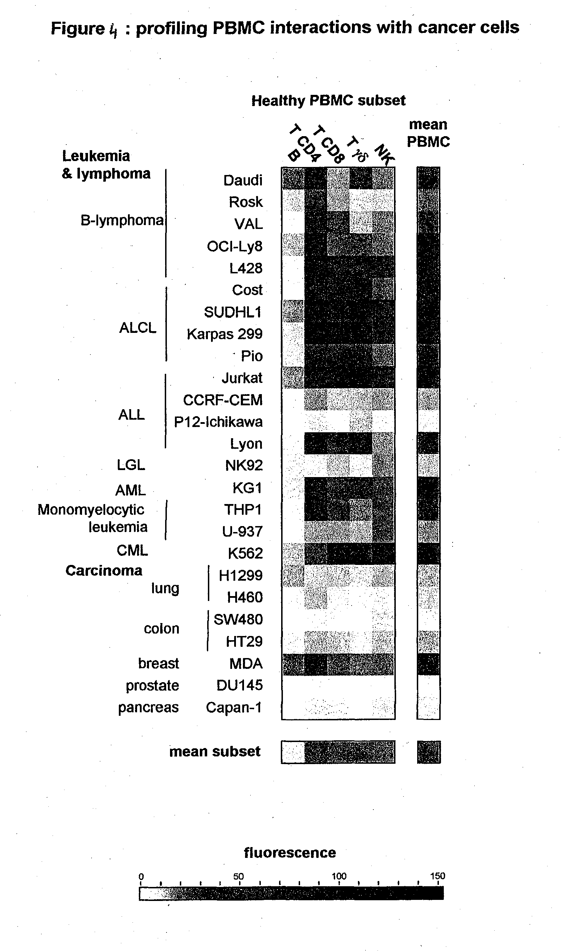

The results are given or Figure 4.

The specified cancer cell lines were tested in parallel as PKH67+ baits in co-incubation with bulk PBMC from donor # A prior to measuring the subset-specific interactions as above. For clarity, the PKH67 mfi for 0 mins co-incubation were not shown (mfi 3-5), and the interaction scores were represented by the specified colour table (see material and methods). This panel was extended to 150 due to the intense interactions with ALCL Karpas-299.

This panel of 25 cell lines encompassed five B-cell lymphoma, four anaplastic large cell lymphoma (ALCL), four acute T cell lymphoblastic leukemia (ALL), one large granular lymphocyte leukemia (LGL), one acute myeloïd leukemia (AML), two myelomonocytic leukemia, one chronic myeloid leukemia (CML), as well as carcinoma from lung, colon, breast, prostate and pancreas.

All cancer cell lines were efficiently labelled with PKH67 (mfi > 5000) and the same fluorescence intensity was recorded for PBMC at t0 of co-culture with each of these targets (mfi =4-6), which is a crucial setting in order to compare only their fluorescence after 60 minutes of co-culture.

The t60 mfi were represented as a colour table of PBMC subsets *versus* cancer cell targets for visualization of the results given on figure 4.

On average with this donor, B lymphocytes consistently gave the weakest scores while the CD4 T cells were the most interactive, and the three "cytolytic" subsets (CD8 T, γδ T and NK) reacted to similar extents to the panel taken as a whole. A global view of these tables constitute an individual cancer reactivity pattern. The shown example underlined both strong interactions with B-cell lymphoma or anaplastic large cell lymphoma and their relative deficiency with most carcinoma.

In this example, the strongest interactions of fresh NK cells were always recorded with K562, in agreement with the NK cell activating phenotype of this erythromyeloid cell line.

With the four non-Hodgkin B-cell lymphoma tested, the most interactive PBMC were CD4 T lymphocytes, while with the Hodgkin B lymphoma cell line L428 both T and NK cells interacted equally well.

Unexpectedly, intense transfers were observed with anaplastic large cell lymphoma, as examplified by T CD4, T CD8 and γδ T lymphocytes co-incubated with Karpas-299 (e.g. mfi 100-150, in three independent experiments).These were the strongest interactions recorded with fresh PBMC, and were found with the four cell lines of this histotype tested in this example.

The results differed with acute lymphoblastic leukemia however, which gave heterogeneous interaction patterns in terms of intensity and effector subsets. Most healthy T and NK cells strongly interacted with Jurkat, but almost no PBMC could interact with P12-Ichikawa.

Whole PBMC from donor #A trogocytosed very weakly most carcinoma cell lines involved in our study, as for instance the prostatic DU145, colon SW480 or pancreas Capan-1 cell lines.

These data demonstrated that even when co-incubated *in vitro,* some cancer cells appear able to avoid contact with functionally unaltered PBMC.

As demonstrated above, because these PBMC interaction patterns differ within human individuals, the global aspect of such tables from each human individual constitutes its respective cancer reactivity pattern.

### Subtyping the γδ T cells interact with anaplastic large cell lymphoma Karpas-299.

Since the innate ability of all γδ T cells to interact with ALCL cell lines such as Karpas-299 was detected above and these cells comprise several different subtypes, similar experiments involving a more refined set of y8 T cell markers have been carried out to prolife further this interaction.

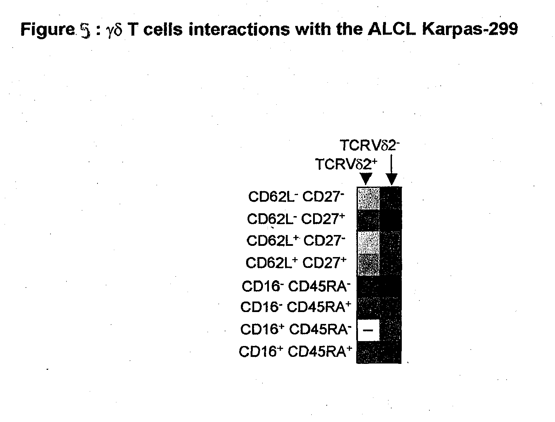

The γδ T cells represented 0.8% of PBMC in donor#A and were essentially composed of TCRVδ2+ lymphocytes (>90% of γδ T cells). Although normal values for healthy human adults, these comprised both naïve and memory TCRVδ2+γδ T lymphocytes.
The results are given in figures 5, which represents the subtyping of the bulk PBMC interacting with Karpas-299 cells *ex vivo*. Only PKH67mfi for 60 mins of co-incubation are shown, using the same colour scale as in Fig. 5 (-: undetected cells).

Fresh PBMC subsets (donor #A) were thus defined here by two parallel combinations of triple stainings for TCRVδ2/CD16/CD45RA and TCRVδ2/CD27/CD62L. Each of these maturation stages were respectively scored for their spontaneous interactions with PKH67+ Karpas-299 cells by the same assay, but through analysis of 100,000 events from the co-culture since they represented only 0.1-2 % of the introduced PBMC. The results identified the most interactive subset of TCRVδ2+ γδ T cells as having the CD16- CD45RA- and CD62L- CD27+ phenotype (Fig. 5). This corresponds to the so-called "central memory" pool of γδ T cells which is thought to preceeds the acquisition of cytolytic activity during their peripheral maturation. The complementary TCRVδ2- PBMC which also scored the highest interactions with Karpas-299 also corresponded to non-naïve central memory cells.

### BIBLIOGRAPHIC REFERENCES

Huang, J. F., Y. Yang, H. Sepulveda, W. Shi, I. Hwang, P. A. Peterson, M. R. Jackson, J. Sprent, and Z. Cai. 1999. TCR-Mediated internalization of peptide-MHC complexes acquired by T cells. *Science 286:952.*
Hudrisier, D., and P. Bongrand. 2002. Intercellular transfer of antigen-presenting cell determinants onto T cells: molecular mechanisms and biological significance. *Faseb J 16:477.*
Joly, E. and Hudrisier, D. 2003.What is trogocytosis and what is its purpose? Nat Immunol, *4*: 815.
Hudrisier, D., J. Riond, H. Mazarguil, J. E. Gairin, and E. Joly. 2001. Cutting edge: CTLs rapidly capture membrane fragments from target cells in a TCR signaling-dependent manner. *J Immunol 166:3645.*
Tabiasco, J., Vercellone, A., Meggetto, F., Hudrisier, D., Brousset, P., and Fournie, J. J. 2003.Acquisition of viral receptor by NK cells through immunological synapse. J Immunol, *170*:5993-5998.
Espinosa, E., Tabiasco, J., Hudrisier, D., and Fournie, J. J. 2002.Synaptic Transfer by Human gammadelta T Cells Stimulated with Soluble or Cellular Antigens. J Immunol, *168:* 6336-6343.
Batista, F. D., D. Iber, and M. S. Neuberger. 2001. B cells acquire antigen from target cells after synapse formation. *Nature 411:489.*
Batista, F. D., and M. S. Neuberger. 2000. B cells extract and present immobilized antigen: implications for affinity discrimination. *Embo J 19:513.*
Poupot, M. and Fournie, J. J. Spontaneous membrane transfer through homotypic synapses between lymphoma cells. J Immunol, *171:* 2517-2523, 2003.

## Claims

1. A method for *in vitro* identifying the living cells from a sample to be tested capable of interacting through trogocytosis with target cells, wherein the living cells consist of or comprise as a major part lymphoid cells and the target cells are labelled, said method comprising detecting the label transferred from the target cells to the living cells, to identify the effector cells.

2. The method of claim 1, comprising
- inserting a fluorescent label at the membrane surface of the target cells,
- co-incubating the fluorescent labelled target cells with said living cells, under conditions enabling the establishment of the interactions between both cellular types in case of trogocytosis, and
- identifying the effector cells, i.e. cells having established such interactions, and quantifying and comparing the amount of transferred label between different types or subsets of living cells, if desired.

3. The method of claim 1 or 2, wherein the target cells correspond to one or several types of mixed cells interacting simultaneously.

4. The method of claim 3, wherein the target cells are cancer cells or cancer cell lines.

5. The method of claim 3 or 4, wherein the insertion of the label is carried out at room temperature and comprises adding a suspension of the target cells in an appropriate medium to a solution containing the label.

6. The method of claim 5, wherein the labels are selected in the group comprising lipophilic fluorochrome, such as PKH67, or membrane protein labelled by an antibody conjugated to a lipophilic fluorochrome.

7. The method of anyone of claims 1 to 6, wherein the living cells to be tested are extracted from biological samples of a patient and correspond to one or more cell population(s), optionally comprising cell subsets.

8. The method of claim 7, wherein the biological samples are blood samples and the living cells extracted therefrom are PBMC or subsets populations of PBMC.

9. The method of anyone of claims 1 to 8, wherein the co-incubation step is carried out during a period of time and under physiological conditions appropriate with the viability of the cells.

10. The method of claim 9, wherein the incubation conditions comprise co-culture of the target cells and the living cells to be tested during at least 1h in a culture medium, at physiological temperature, under humidified 5% CO₂ in air atmosphere.

11. The method of anyone of claims 1 to 10, wherein the co-cultures are further labelled with specific antibodies conjugated to a fluorochrome having an emission wavelength distinct from the one of the targets.

12. The method of anyone of claims 1 to 11, wherein simultaneous tests of several targets and effectors are performed in parallel, in single test tubes and wells or multiwell plates and an automated sampling and polychromatic flow cytometry analyzer is used.

13. The method of anyone of claims 1 to 11, wherein multiple experiments are performed simultaneously or in parallel in the same culture well or flask or mutliwell plates, by placing in each co-culture several types of target cells, each of which are respectively labelled with specific antibodies conjugated to fluorochromes with a different emission wavelength spectra, and using an automated polychromatic flow cytometry analyzer.

14. The method of anyone of claims 1 to 13, wherein the quantification step of the dual fluorescence intensities on the effector cells is advantageously carried out by flow cytometry.

15. The method of anyone of claims 1 to 14, wherein the target cell is not a living cell or is a solid surface covered by a material capable of interacting with said living cells.

16. Kits for carrying out the method according to anyone of claims 1 to 15, comprising cell labels, particularly lipophilic fluorochromes for the labelling of cell targets and antibodies.

17. Kits according to claim 16, further comprising culture buffers and/or rinsing buffers.

18. Kits according to claim 16 or 17, further comprising target cells and/or effector cells, and/or culture supports.

19. The use of the method according to anyone of claims 1 to 15 or the kits according to anyone of claims 16 to 18 in hematology, oncology and immunology.
